# EUROPÄISCHE PATENTSCHRIFT

(11) **EP 1 702 565 B1**
(45) Veröffentlichungstag und Bekanntmachung des Hinweises auf die Patenterteilung: **21.04.2010**
(21) Anmeldenummer: 06004546.5
(22) Anmeldetag: 07.03.2006
(51) Int. Cl.: A61B 5/151, A61B 5/00, G01N 33/487, G01N 35/00, A61B 10/00

(54) **Bandmagazin für ein Handgerät zur Untersuchung einer Körperflüssigkeit, sowie Handgerät**
Strip magazine for hand-held device for analyzing body fluids, and hand-held device
Magasin-ruban pour un dispositif d'essai portatif pour examiner des liquides organiques, et dispositif d'essai portatif

(30) Priorität: 18.03.2005 DE 102005013685
(43) Veröffentlichungstag der Anmeldung: 20.09.2006
(60) Teilanmeldung: 10156032.4
(73) Patentinhaber: F.HOFFMANN-LA ROCHE AG, 4070 Basel (CH); Roche Diagnostics GmbH, 68305 Mannheim (DE)
(72) Erfinder: List, Hans, 64754 Hesseneck-Kailbach (DE); Haar, Hans-Peter, 69168 Wiesloch (DE); Roesicke, Bernd, 68305 Mannheim (DE)
(74) Vertreter: Pfiz, Thomas

(56) Entgegenhaltungen:
- WO-A-2004/056269
- WO-A-2004/057345
- FR-A1- 2 565 350
- US-A1- 4 218 421

## Beschreibung

Die Erfindung betrifft ein Bandmagazin für ein Handgerät zur Untersuchung einer Körperflüssigkeit mit einem Testband, einer Vorratseinheit für unverbrauchtes Testband und einer Abfalleinheit für verbrauchtes Testband, wobei die Abfalleinheit zum Vorspulen des Testbandes angetrieben ist. Solche Bandmagazine sind z.B. aus WO-A-2004/057345, WO-A-2004/056269 bekannt.

Bei derartigen Bandmagazinen ist es vorgesehen, dass von einem Vorratswickel ein unbenutzter Testbandabschnitt abgezogen und über eine Aufnahmeeinrichtung geführt wird, wo er eine Probe der Körperflüssigkeit aufnimmt. Anschließend wird der nun benutzte Testbandabschnitt auf einem Abfallwickel aufgewickelt. Der Aufnahmeeinrichtung ist eine Detektionseinrichtung zugeordnet, welche die Probe vermisst und das Messergebnis an eine Auswerteeinrichtung weiterleitet.

Bevorzugte werden solche Bandmagazine in Blutzuckermessgeräten für Diabetiker eingesetzt, die auf eine ständige Kontrolle ihrer Blutzuckerwerte angewiesen sind. Das Testband ermöglicht nach dem Applizieren von Kapillar-blut, beispielsweise aus der Fingerbeere, einen geräteinternen Blutzuckernachweis. Hierfür ist eine Vielzahl von Testabschnitten bzw. Testfeldern auf dem Testband fortlaufend angeordnet. Ein unverbrauchter Testabschnitt wird durch einen Bandvorlauf in eine aktive Position gebracht. Dann wird das Kapillarblut appliziert und analysiert. Für eine einfache Dosierung geringster Blutmengen und für eine möglichst genaue Positionierung bezüglich der Detektionseinrichtung wird das Testband über eine gehäuseinterne Umlenkspitze gelenkt. Dabei besteht die Gefahr von Fehlmessungen, wenn das Testband von der Umlenkspitze herunterrutscht. Für eine erfolgreiche Messung muss das Testband an einer wohldefinierten Stelle verbleiben und glatt anliegen. Dabei sollte ein vorbestimmter Abstand zur Detektionseinrichtung eingehalten werden. Dies gilt mindestens so lange, bis die Messung abgeschlossen ist. Eine weitere Herausforderung liegt darin, dass das Testband sehr empfindlich gegen Kontamination ist. Der unbenutzte Bereich des Testbandes sollte daher gegenüber dem benutzten Bereich räumlich getrennt und auch von äußeren Einflüssen abgeschirmt werden, welche die Funktion des Testbandes beeinträchtigen könnten. Eine direkte Antriebskopplung zwischen der Vorratseinheit und der Abfalleinheit ist daher nur schwer möglich.

Ferner sind die vorbekannten Handgeräte für einen Dauergebrauch vorgesehen, während das Bandmagazin ausgewechselt wird. Die Handgeräte sind daher relativ groß und, nicht zuletzt wegen der aufwendigen Gerätetechnik, recht fertigungsintensiv.

Die Aufgabe der vorliegenden Erfindung besteht somit darin, ein Bandmagazin bereit zu stellen, bei welchem eine Fehlfunktion durch eine Bandlose vermieden wird.

Die Aufgabe besteht ferner darin, ein Handgerät in kompakter und herstellungstechnisch günstiger Bauform bereit zu stellen.

Zur Lösung dieser Aufgabe wird die in dem unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Dementsprechend wird vorgeschlagen, dass im Bandmagazin Bremsmittel integriert sind, welche das Testband unter Zugspannung halten. Damit kann der erforderliche Mindestbandzug aufrecht erhalten werden, da der Antrieb an der Abfalleinheit das Testband gegen diese Zugspannung transportiert, so dass es nicht zu weit von der Vorratseinheit abgezogen oder gar ungewollt abgespult wird. Auf diese Weise kann das Testband sicher und in einer wohldefinierten Position in Bezug auf die Detektionseinheit umgelenkt werden. Der Mindestbandzug verhindert dabei auch ungewollte Seitenverschiebungen. In jedem Fall sollte die Bandantriebskraft ausreichend bemessen sein, um die Wirkung des Bremsmittels überwinden zu können.

Es ist insbesondere von Vorteil, wenn die Vorratseinheit mit einer Bremskraft beaufschlagt ist, welche das Testband unter Zugspannung hält. Damit wird insbesondere vermieden, dass verbrauchtes Testband ungewollt in Richtung auf die Applikationsstelle abspult. Ferner wird verhindert, dass bereits mit Blut verunreinigte Bereiche des Testbandes wieder aus dem Bandmagazin herausgezogen werden, was einen wichtigen hygienischen Vorteil darstellt.

Die Bremsmittel können unmittelbar auf das Testband eine Bremskraft ausüben. Alternativ ist es möglich, dass die Bremsmittel an einem Spulenkörper für das Testband angreifen, so dass das Testband mittelbar gebremst ist. Im letzteren braucht ein veränderlicher Durchmesser des Bandwickels nicht berücksichtigt zu werden. Außerdem wird dadurch das Testband nicht zusätzlich mechanisch beansprucht.

In einer einfachen Ausführung ist es vorgesehen, dass die Bremsmittel eine konstante Bremskraft auf die Vorratseinheit ausüben. Dies lässt sich dadurch realisieren, dass die Bremsmittel als Reibelement ausgebildet sind und insbesondere eine Blattfeder als Reibelement aufweisen. Ein Zusatznutzen wird dadurch erreicht, dass die Bremsmittel durch eine Dichtung zur Abdichtung der Vorratseinheit an einem Durchlass für das Testband gebildet sind.

In einer etwas aufwändigeren Ausführungsform wirken die Bremsmittel mit variabler Bremskraft auf die Vorratseinheit ein. Damit kann erreicht werden, dass die benötigte Kraft zum Abziehen des Testbands mit abnehmendem Durchmesser des Vorratswickels gegenüber der oben beschriebenen einfachen Ausführung weniger stark ansteigt. In diesem Zusammenhang ist zu berücksichtigen, dass der Vorratswickel eine gewisse Lagerreibung aufweist, die durch den Antrieb überwunden werden muss. Dementsprechend muss ein Drehmoment aufgebracht werden, welches sich aus dem augenblicklichen Radius des Abfallwickels und der aufgebrachten Kraft zum Abspulen des Bandes vom Vorratswickel ergibt. Wenn also der Radius des Vorratswickels mit der Zeit abnimmt, muss diese Kraft ansteigen. Wird hierbei die Bremskraft in Abhängigkeit vom Bandzug verringert, kann insgesamt der Kraftanstieg geringer gehalten werden.

Eine vorteilhafte Ausführung sieht vor, dass die Bremsmittel einen vorzugsweise über eine Feder mit einer Bremskraft belasteten Umlenkhebel aufweisen, wobei der Umlenkhebel das Testband vorzugsweise über eine Rolle umlenkt. Dabei findet mit zunehmender Bandzugkraft eine Entlastung des Umlenkhebels statt. Der Umlenkhebel kann beispielsweise mit einer Blattfeder oder Druckfeder zur Ausübung der Bremskraft belastet sein und wirkt vorzugsweise auf ein Spulengehäuse für das Testband als Bremse.

Eine weitere Verbesserung sieht vor, dass die Bremskraft nicht nur durch den Bandzug, sondern mit Hilfe eines Kompensationsmechanismus auch durch den aktuellen Wickeldurchmesser moduliert wird, so dass der Bandzug nahezu konstant bleibt.

Dieses Ziel kann mit einem Kompensationsmechanismus erreicht werden, der eine den Bandwickel umfangsseitig abtastende federbelastete Schwinge aufweist, wobei mit abnehmendem Durchmesser des Bandwickels die Federbelastung abnimmt und dementsprechend die Bremskraft reduziert wird. Die Bandzugkraft bleibt somit konstant.

An der Abfalleinheit ist eine formschlüssig oder kraftschlüssig wirkende Rücklaufsperre oder Rücklaufbremse vorgesehen. Bei der Rücklaufsperre als Sicherungsmittel braucht der Antrieb zum Vorspulen des Testbandes nur die Reibung der Vorratseinheit zu überwinden, was insbesondere die Batterie des Antriebs schont. Diese Rücklaufsicherung sollte vorteilhafterweise nicht auf den Bandantrieb wirken, so dass sie auch bei einer Entnahme des Bandmagazins aus dem Handgerät wirksam ist und ein ungewolltes Ausspulen von gebrauchtem Band vermieden wird. Die Rücklaufsperre kann in an sich bekannter Weise als Gesperre ausgebildet sein, welches in eine Verzahnung beispielsweise im Spulengehäuse der Abfalleinheit so eingreift, dass ein Formschluss in Gegenlaufrichtung ein Zurückdrehen der Abfalleinheit verhindert. Die Abfalleinheit ist dann auf die Drehrichtung "Aufwickeln" beschränkt. Es kann aber auch ein kraftschlüssig wirkendes Gesperre vorgesehen sein. Hierfür sind verschiedene Mechanismen denkbar, beispielsweise eine Schlingfedersicherung oder ein Klemmrollenfreilauf.

Das Bandmagazin weist vorzugsweise ein das Testband, die Vorratseinheit und die Abfalleinheit umschließendes Gehäuse auf. Die Vorratseinheit sollte hierbei in einem Vorratsraum aufgenommen sein, der gegen das Testband beeinträchtigende Einflüsse abgeschirmt ist. Dies kann beispielsweise dadurch erreicht werden, dass eine Wand des Vorratsraums einen überlappenden Bereich mit einer Wand des Gehäuses bildet, wobei entlang des überlappenden Bereichs ein Durchlass für das Testband ausgebildet ist. Dieser sollte vorteilhafterweise mit mindestens einem Dichtmittel versehen sein, um das Testband vor äußeren Einflüssen zu schützen.

Im Folgenden wird die Erfindung anhand der in der Zeichnung schematisch dargestellten Ausführungsbeispiele näher erläutert. Es zeigen:
- Fig. 1: ein erstes Ausführungsbeispiel eines erfindungsgemäßen Bandmagazins im Schnitt;
- Fig. 2: ein zweites Ausführungsbeispiel eines erfindungsgemäßen Bandmagazins in einer teilweise geschnittenen Seitenansicht;
- Figur 3: ein drittes Ausführungsbeispiel eines erfindungsgemäßen Bandmagazins in einer teilweise geschnittenen Seitenansicht;
- Figur 4: ein Ausführungsbeispiel eines mechanisch betriebenen Bandgeräts in einer perspektivischen Darstellung mit teilweise geöffnetem Gehäuse;
- Figur 5: das Gerät gemäß Figur 4 mit geschlossenem Gehäuse.

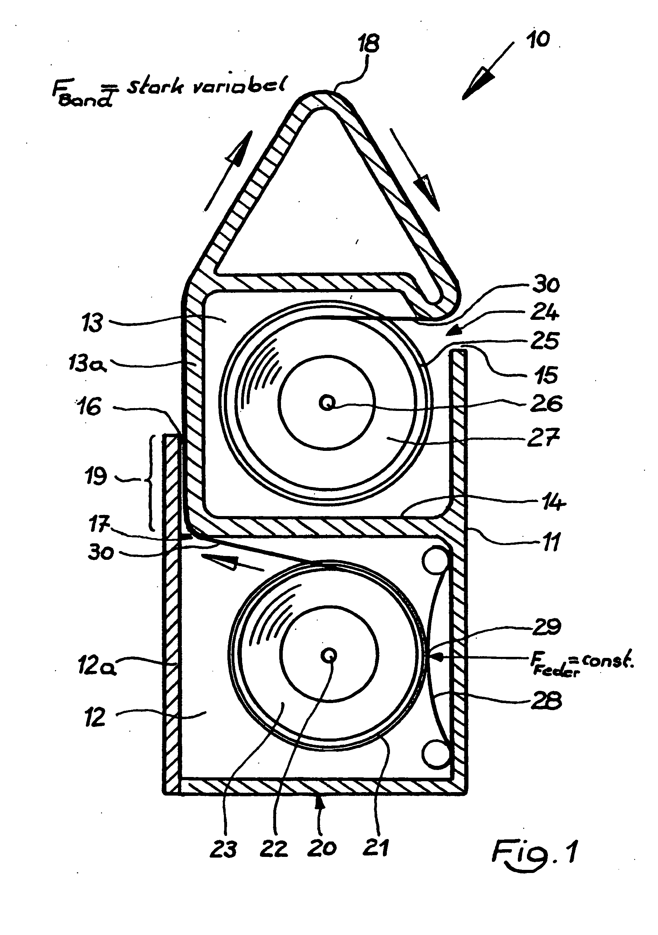

Die Figuren 1 bis 3 zeigen verschiedene Ausführungsbeispiele eines erfindungsgemäßen Bandmagazins, bei dem das Testband unter Zugspannung gehalten ist. Figur 1 zeigt ein Bandmagazin 10 mit einem Gehäuse 11. Das Gehäuse 11 ist unterteilt in eine erste Aufnahme 12 für eine Vorratseinheit 20 und eine zweite Aufnahme 13 für eine Abfalleinheit 24. Die Aufnahmen 12, 13 sind durch eine Trennwand 14 voneinander getrennt, so dass die Vorratseinheit 20 von der Abfalleinheit 24 separiert ist. Je eine Seitenwand 12a, 13a der Aufnahmen 12, 13 überlappen sich im Bereich 19 und bilden eine Öffnung 16, die mit einer Dichtung 17 versehen ist. Eine weitere Öffnung 15 ist in der Aufnahme 13 für die Abfalleinheit 24 vorgesehen. In das Gehäuse 10 ist eine Umlenkspitze 18 für das Testband 30 integriert.

Die Vorratseinheit 20 weist ein Spulengehäuse 21 auf, welches ein um eine Spule 22 zu einem Vorratswickel 23 aufgewickeltes unverbrauchtes Testband 30 aufnimmt. In vergleichbarer Weise ist die Abfalleinheit 24 mit einem Spulengehäuse 25 ausgestattet, welches das um eine Spule 26 zu einem Abfallwickel 27 aufgewickelte verbrauchte Testband aufnimmt. Die Spule 26 wird von einem Antrieb (nicht dargestellt) angetrieben. Das Testband 30 ist in hintereinander liegende Testbereiche aufgeteilt. Wird der Antrieb betätigt, so wird das frische Testband 30 vom Vorratswickel 23 abgespult und aus der Aufnahme 12 durch die Öffnung 16 hindurch zu der Umlenkspitze 18 geführt, wo ein einzelner Testbereich frei nach außen zu liegen kommt und eine Testflüssigkeit, beispielsweise einen Blutstropfen, aufnehmen kann. Die Testflüssigkeit wird von einer Detektionseinheit (nicht dargestellt) vermessen. Bei nochmaligem Betätigen des Antriebs wird das Testband 30 weiter transportiert. Das verbrauchte Testband 30 tritt durch die Öffnung 15 in die Aufnahme 13 ein und wird auf dem Abfallwickel 27 aufgewickelt.

An einer Innenwand der Aufnahme 12 für die Vorratseinheit 20 ist eine Blattfeder 28 vorgesehen, die am Kontaktpunkt 29 das Spulengehäuse 21 beaufschlagt. Die Blattfeder 28 ist mit einer konstanten Federkraft F_{Feder} vorgespannt. Daher muss das Testband 30 gegen das entsprechende Bremsmoment mit einer gewissen Bandzugkraft F_{Band} abgespult werden. Diese Bandzugkraft nimmt mit abnehmendem Radius des Vorratswickels 23 zu.

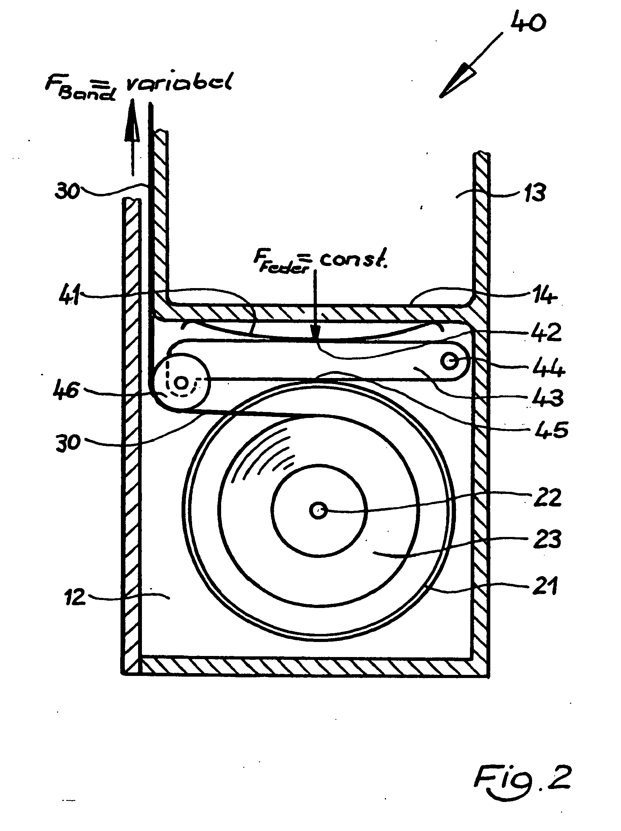

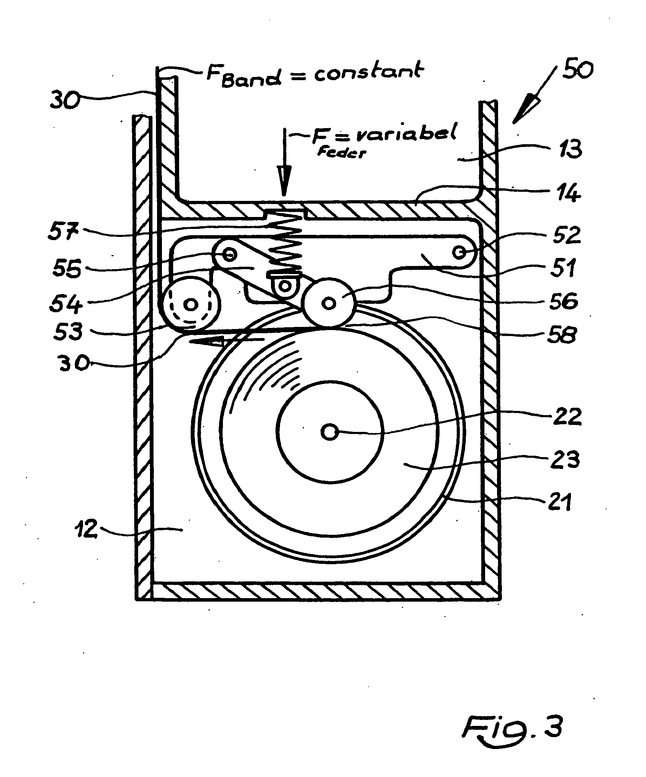

Die Figuren 2 und 3 zeigen zwei weitere Ausführungsbeispiele eines erfindungsgemäßen Bandmagazins 40, 50, welche sich von dem soeben beschriebenen Bandmagazin 10 nur in der Ausgestaltung der Bremsmittel unterscheiden. Gleiche Bauteile sind daher mit gleichen Bezugszeichen versehen.

Das in Figur 2 gezeigte Bandmagazin 40 weist an einer Innenwand der Aufnahme 12 unterhalb der Trennwand 14 ebenfalls eine Blattfeder 41 auf, die mit einer konstanten Federkraft F_{Feder} vorgespannt ist. An der Innenwand der Aufnahme 12 ist ferner ein Schwinghebel 43 um eine Achse 44 drehbar angelenkt. Der Schwinghebel 43 ist an seinem freien Ende mit einer drehbaren Umlenkrolle 46 versehen, über welche das Testband 30 geführt ist. Der Schwinghebel 43 ist zwischen der Blattfeder 41 und dem Spulengehäuse 21 angeordnet und verläuft tangential zum Spulengehäuse 21. Der Schwinghebel 43 berührt die Blattfeder 41 in einem Kontaktpunkt 42 und das Spulengehäuse 21 in einem Kontaktpunkt 45. Der Schwinghebel 43 wird von der Blattfeder 41 mit der konstanten Federkraft F_{Feder} beaufschlagt. Demzufolge wird auch das Spulengehäuse 21 vom Schwinghebel 43 mit einer entsprechenden Kraft beaufschlagt.

Wenn der Antrieb für das Testband 30 betätigt wird, dann muss es gegen das auf das Spulengehäuse 21 wirkende Bremsmoment mit einer gewissen Bandzugkraft F_{Band} abgespult werden. Die Bandzugskraft greift am langen Hebelarm des Schwinghebels 43 über die Umlenkrolle 46 an und entlastet den Kontaktpunkt 45 abhängig von der Bandzugkraft. Mit abnehmendem Durchmesser des Vorratswickels 23 muss also eine geringere Bandzugkraft zum Abspulen des Testbands aufgebracht werden als bei dem in Figur 1 dargestellten Ausführungsbeispiel.

Das in Figur 3 dargestellte Ausführungsbeispiel eines Bandmagazins 50 weist an der Innenwand der Aufnahme 12 unterhalb der Trennwand 14 ebenfalls einen Schwinghebel 51 auf, der um eine Achse 52 drehbar an der Innenwand angelenkt ist. Der Schwinghebel 51 ist an seinem freien Ende auch mit einer drehbaren Umlenkrolle 53 versehen, über welche das Testband 30 geführt ist. Der Schwinghebel 51 verläuft ebenfalls tangential zum Spulengehäuse 21 und berührt das Spulengehäuse 21 in einem hier nicht sichtbaren Kontaktpunkt.

Am Schwinghebel 51 ist eine um eine Achse 55 drehbare Schwinge 54 angelenkt. Die Schwinge 54 ist an ihrem freien Ende mit einer Abtastrolle 56 versehen, welche auf dem Umfang des Vorratswickels 23 an einem Kontaktpunkt 58 aufliegt. Eine mit einer bestimmten Federkraft F_{Feder} vorgespannte Druckfeder 57 stützt sich an der Trennwand 14 und an der Schwinge 54 ab, so dass die Schwinge 54 mit dieser Federkraft beaufschlagt ist.

Wenn der Antrieb für das Testband 30 betätigt wird, dann muss es gegen die auf das Spulengehäuse 21 wirkende Kraft mit einer gewissen Bandzugkraft F_{Band} abgespult werden. Diese Bandzugskraft greift am langen Hebelarm des Schwinghebels 51 an und entlastet den Kontaktpunkt zwischen dem Schwinghebel 51 und dem Spulengehäuse 21 abhängig von der Bandzugkraft. Gleichzeitig läuft die Abtastrolle 56 der Schwinge 54 auf dem Umfang des Vorratswickels 23. Mit abnehmendem Radius des Vorratswickels 23 wandert der Abtastrolle 56 in Richtung der Spule 22, so dass die Druckfeder 57 sich mit abnehmendem Radius des Vorratswickels 23 entspannt. Damit wird der Kontaktpunkt zwischen dem Schwinghebel 51 und dem Spulengehäuse 21 in Abhängigkeit vom Radius des Vorratswickels 23 entlastet. Als Resultat bleibt die vom Antrieb aufzubringende Bandzugskraft F_{Band} mit abnehmendem Radius des Vorratswickels 23 konstant.

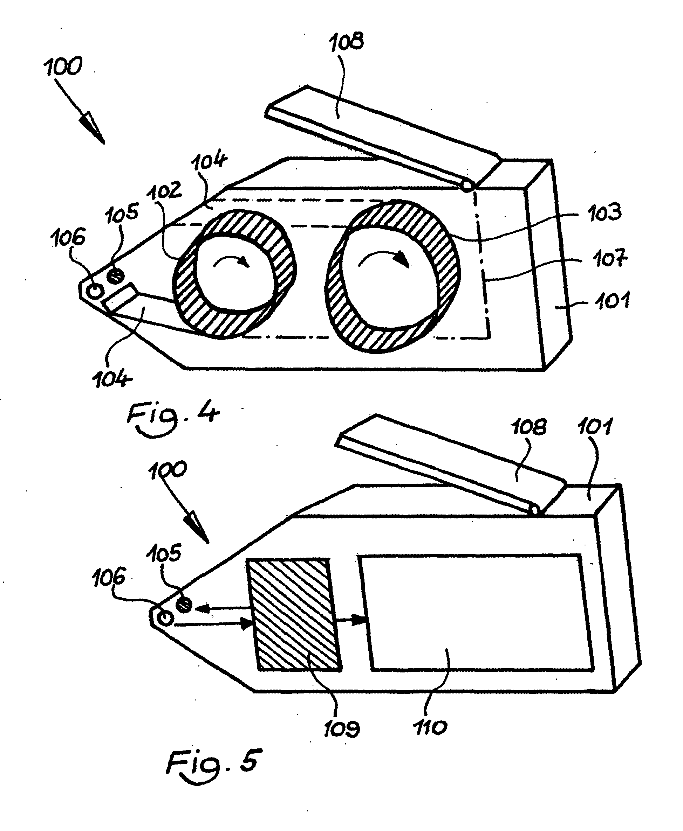

Die Figuren 4 und 5 zeigen eine Kombination aus einem Handgerät mit elektronischen Bauteilen auf Polymerbasis und einem mechanischen Antrieb für das Testband.

Das Handgerät 100 ist ein Einweggerät, ein sogenanntes Disposable. Es weist ein Gehäuse 101 aus Kunststoff auf, in welchem zwei Spulenkörper 102, 103 angeordnet sind. Auf den Spulenkörpern 102, 103 ist ein Testband 104 mit aufeinander folgenden Testfeldern aufgewickelt. Im Gehäuse 101 ist ferner ein photooptischer Sensor 105 in räumlicher Nähe zu einem Messort 106 vorgesehen. Am Messort 106 ist das Testband 104 von außen zugänglich, um eine Probenflüssigkeit, beispielsweise Blut zur Blutzuckerbestimmung, aufzunehmen. Der Bereich zwischen Sender und Empfänger des Sensors und dem Testband kann durch optischen Strahlengang oder Lichtleiter überbrückt werden. Der vom Sensor 105 aufgenommene Messwert wird in eine Auswerteeinheit 109 übertragen. Dort wird ein Anzeigewert, beispielsweise der Blutzuckergehalt, berechnet.

Der photooptische Sensor besteht aus mindestens einer LED geeigneter Wellenlänge, vorzugsweise OLED, kombiniert mit einer oder mehreren organischen Photodioden (Multiphotometerprinzip). Auch LEDs mit Mehrfachwellenlängen sind denkbar.

Die Auswerteeinheit 109 enthält beispielsweise Verstärker, AD-Konverter, Rechenwerk, Schaltwerk, Datenspeicher, Energieversorgung und Schnittstellen und ist mit einer Anzeigeeinheit 110 verbunden, die den ermittelten Anzeigewert auf einem Display darstellt. Die Anzeigeeinheit kann in an sich bekannter Weise so ausgebildet sein, dass eine Anzeige bis zum nächsten Messvorgang auch ohne Energieversorgung aufrechterhalten werden kann, beispielsweise durch Verwendung sog. "elektronischer Tinten".

Der Datenspeicher in der Auswerteeinheit kann als ROM oder EEPROM ausgebildet sein. Er wird hauptsächlich zur Speicherung chargenspezifischer Daten benötigt, die bei der Produktion des Disposables ermittelt und darauf hinterlegt werden. Die Datenübermittlung geschieht mittels Kontaktschnittstellen oder RF-ID-Transponder. Mit einem EEPROM wäre auch ein elektronisches Testfeld-Zählwerk realisierbar.

Die elektronischen Bauteile dieses Handgeräts sind an sich bekannte polymerelektronische Bauteile. Solche Bauteile sind in der WO 2004/044571 A1 beschrieben. Die Verwendung derartiger Bauteile ermöglicht es, sämtliche notwendigen elektronischen Komponenten in ein Magazingehäuse zu integrieren, so dass das resultierende Bandmagazin zugleich ein voll funktionsfähiges und sehr handliches Einweg-Handgerät darstellt. Ein derartiges Einweg-Handgerät ist klein und leicht, kostengünstig und einfach zu bedienen. Das Wechseln des Bandmagazins entfällt. Der Weg für eine weitergehende Miniaturisierung von tragbaren Handgeräten ist offen. Die aufwendige Konstruktion von Schnittstellen zwischen Bandmagazin und Handgerät entfällt ebenfalls.

Alle elektronischen Bauteile auf Polymerbasis können in an sich bekannter Weise auf geeignete Ausformungen des Gehäuses 101 des Handgeräts 100 aufgedruckt werden.

Die Energieversorgung erfolgt mit Hochleistungskondensatoren (Supercap), beispielsweise kombiniert mit Solarzellen. Aufgrund der geringen erreichbaren Energiedichte empfiehlt es sich, die Spulenkörper 102, 103 des beschriebenen Einweg-Handgeräts manuell anzutreiben.

Die Spulenkörper 102, 103 sind hierfür mit Zähnen oder Stufen versehen. In diese Zähne oder Stufen greift eine hier nur angedeutete Vorschubklinke 107 ein. Die Vorschubklinke 107 ist mit einem außen am Gehäuse vorgesehenen Hebel 108 verbunden. Der Vorschub des Testbandes 104 erfolgt durch Betätigen des Hebels. Dabei werden die Spulenkörper 102, 103 genau so weit bewegt, dass ein frisches Testfeld des Testbandes 104 am Messort 106 von außen zugänglich ist. Zur Synchronisation der Bewegungen von Spulenkörpern 102, 103 und Testband 104 ist letzteres perforiert, so dass auf den Spulenkörpern 102, 103 angeordnete Zähne (nicht dargestellt) in die Perforation eingreifen. Die Testfelder können auch so auf dem Testband beabstandet verteilt sein, dass bei einer ersten Betätigung des Hebels 108 ein frisches Testfeld am Messort 106 zugänglich gemacht wird. Mit einer zweiten Betätigung des Hebels 108 wird das nun verbrauchte Testfeld vom Messort 106 weg bewegt, ohne dass dabei sofort ein neues Testfeld erscheint. Dies geschieht erst bei einer neuerlichen Betätigung des Hebels 108.

Mit der Betätigung des Hebels 108 kann auch die für die Messung notwendige Energie von wenigen Milliwatt in an sich bekannter Weise generiert und beispielsweise in einem Kondensator bzw. Supercap zwischengespeichert werden. Zur generatorischen oder piezoelektrischen Energiegewinnung kann ein mechanischer Zwischenspeicher in Form einer Feder vorgesehen sein, der es erlaubt, die unterschiedlichen Zeitkonstanten anzupassen.

Durch Verzicht auf einen elektrochemischen Energiespeicher wird ein besonders umweltfreundliches Gerätekonzept realisierbar.

## Patentansprüche

1. Bandmagazin für ein Handgerät (100) zur Untersuchung einer Körperflüssigkeit mit einem Testband (30), einer Vorratseinheit (20) für unverbrauchtes Testband und einer Abfalleinheit (24) für verbrauchtes Testband, wobei die Abfalleinheit (24) zum Vorspulen des Testbandes (30) angetrieben ist, und mit integrierten Bremsmitteln (17,28,43,51), welche das Testband (30) unter Zugspannung halten, **dadurch gekennzeichnet, dass** die Abfalleinheit (24) eine Rücklaufsperre gegen ungewolltes Bandabwickeln aufweist.

2. Bandmagazin nach Anspruch 1, **dadurch gekennzeichnet, dass** die Vorratseinheit (20) mit einer Bremskraft beaufschlagt ist.

3. Bandmagazin nach Anspruch 1 oder 2, **dadurch gekennzeichnet,** die Bremsmittel (17,28,43,51) unmittelbar auf das Testband (30) eine Bremskraft ausüben.

4. Bandmagazin nach Anspruch 1 oder 2, **dadurch gekennzeichnet,** die Bremsmittel (17,28,43,51) an einem Spulenkörper (21) für das Testband (30) angreifen, so dass das Testband mittelbar gebremst ist.

5. Bandmagazin nach einem der Ansprüche 1 bis 4, **dadurch gekennzeichnet, dass** die Bremsmittel (17,28,43,51) eine konstante Bremskraft auf die Vorratseinheit (20) ausüben.

6. Bandmagazin nach einem der Ansprüche 1 bis 5, **dadurch gekennzeichnet, dass** die Bremsmittel (17,28,43,51) als Reibelement ausgebildet sind.

7. Bandmagazin nach einem der Ansprüche 1 bis 6, **dadurch gekennzeichnet, dass** die Bremsmittel (17,28,43,51) eine Blattfeder (28) als Reibelement aufweisen.

8. Bandmagazin nach einem der Ansprüche 1 bis 6, **dadurch gekennzeichnet, dass** die Bremsmittel (17,28,43,51) durch eine Dichtung (17) zur Abdichtung der Vorratseinheit (20) an einem Durchlass (16) für das Testband (30) gebildet sind.

9. Bandmagazin nach einem der Ansprüche 1 bis 4, **dadurch gekennzeichnet, dass** die Bremsmittel (17,28,43,51) mit variabler Bremskraft auf die Vorratseinheit (20) einwirken.

10. Bandmagazin nach Anspruch 9, **dadurch gekennzeichnet, dass** die Bremskraft abhängig vom Durchmesser eines Bandwickels (23) der Vorratseinheit (20) oder abhängig von der Bandzugkraft beim Vorspulen veränderlich ist.

11. Bandmagazin nach Anspruch 9 oder 10, **dadurch gekennzeichnet, dass** die Bremsmittel (17,28,43,51) einen vorzugsweise über eine Feder (41) mit einer Bremskraft belasteten Umlenkhebel (43,51) aufweisen, wobei der Umlenkhebel das Testband (30) vorzugsweise über eine Rolle (46,53) umlenkt.

12. Bandmagazin nach einem der Ansprüche 9 bis 11, **dadurch gekennzeichnet, dass** die Bremsmittel (17,28,43,51) einen Kompensationsmechanismus (54) aufweisen, welcher mit einer Kraft belastet ist, die abhängig vom Durchmesser eines Bandwickels (23) der Vorratseinheit (20) veränderlich ist.

13. Bandmagazin nach Anspruch 12, **dadurch gekennzeichnet, dass** der Kompensationsmechanismus durch eine den Bandwickel (23) umfangsseitig berührende federbelastete Schwinge (54) gebildet ist, wobei mit abnehmendem Durchmesser des Bandwickels (23) die Federbelastung abnimmt.

14. Bandmagazin nach einem der Ansprüche 1 bis 13, **dadurch gekennzeichnet, dass** die Rücklaufsperre als formschlüssig wirkendes Formgesperre oder als kraftschlüssig wirkendes Reibgesperre, insbesondere als Schlingfedersicherung oder Klemmrollenfreilauf, ausgebildet ist.

15. Bandmagazin nach einem der Ansprüche 1 bis 14, **dadurch gekennzeichnet, dass** die Rücklaufsperre in eine Verzahnung beispielsweise im Spulengehäuse der Abfalleinheit so eingreift, dass ein Formschluss in Gegenlaufrichtung ein Zurückdrehen der Abfalleinheit verhindert.

16. Bandmagazin nach einem der vorhergehenden Ansprüche, **dadurch gekennzeichnet, dass** die Vorratseinheit (20) durch einen Gehäuseteil (12) gegen das Testband (30) beeinträchtigende Einflüsse abgeschirmt ist.

17. Bandmagazin nach einem der vorhergehenden Ansprüche, **dadurch gekennzeichnet, dass** eine Gehäusewand (12a) einen überlappenden Bereich (19) mit einer Gehäusewand (13a) bildet, wobei entlang des überlappenden Bereichs (19) ein vorzugsweise mit einem Dichtmittel (17) zur Abdichtung gegenüber dem Testband (30) versehener Durchlass (16) für das Testband (30) ausgebildet ist.

18. Handgerät zur Untersuchung einer Körperflüssigkeit, insbesondere ein tragbares Blutzuckermessgerät, mit einem Bandmagazin (10, 40, 50) nach einem der Ansprüche 1 bis 17, welches vorzugsweise auswechselbar ist.

## Claims

1. Tape magazine for a hand-held device (100) for analysing a body fluid comprising a test tape (30), a storage unit (20) for unused test tape and a waste unit (24) for used test tape where the waste unit (24) is driven in order to wind forward the test tape (30), and comprising integrated braking means (17, 28, 43, 51) which hold the test tape (30) under tension, **characterized in that** the waste unit (24) has a recoil lock to prevent unintentional tape unreeling.

2. Tape magazine according to claim 1, **characterized in that** a braking force is applied to the storage unit (20).

3. Tape magazine according to claim 1 or 2, **characterized in that** the braking means (17, 28, 43, 51) exert a direct braking force on the test tape (30).

4. Tape magazine according to claim 1 or 2, **characterized in that** the braking means (17, 28, 43, 51) act on a reel body (21) for the test tape (30) such that the test tape is indirectly braked.

5. Tape magazine according to one of the claims 1 to 4, **characterized in that** the braking means (17, 28, 43, 51) exert a constant braking force on the storage unit (20).

6. Tape magazine according to one of the claims 1 to 5, **characterized in that** the braking means (17, 28, 43, 51) are designed as a frictional element.

7. Tape magazine according to one of the claims 1 to 6, **characterized in that** the braking means (17, 28, 43, 51) have a leaf spring (28) as frictional element.

8. Tape magazine according to one of the claims 1 to 6, **characterized in that** the braking means (17, 28, 43, 51) are formed by a seal (17) to seal the storage unit (20) at a passage (16) for the test tape (30).

9. Tape magazine according to one of the claims 1 to 4, **characterized in that** the braking means (17, 28, 43, 51) act on the storage unit (20) with a variable braking force.

10. Tape magazine according to claim 9, **characterized in that** the braking force is variable depending on the diameter of a tape spool (23) of the storage unit (20) or depending on the tape tensile force when winding forwards.

11. Tape magazine according to claim 9 or 10, **characterized in that** the braking means (17, 28, 43, 51) have a deflector lever (43, 51) which is loaded with a braking force preferably via a spring (41) where the deflector lever deflects the test tape (30) preferably over a roller (46, 53).

12. Tape magazine according to one of the claims 9 to 11, **characterized in that** the braking means (17, 28, 43, 51) have a compensation mechanism (54) which is loaded with a force that changes depending on the diameter of a tape spool (23) of the storage unit (20).

13. Tape magazine according to claim 12, **characterized in that** the compensation mechanism is formed by a spring-loaded rocker arm (54) which touches the circumference of the tape spool (23) whereby the spring loading decreases as the diameter of the tape spool (23) decreases.

14. Tape magazine according to one of the claims 1 to 13, **characterized in that** the recoil lock is formed as a form locking mechanism which acts in a form-fitting manner, or as a friction locking mechanism, in particular a wrap spring lock or a clamp roller free-wheel, which acts in a frictional manner.

15. Tape magazine according to claim 14, **characterized in that** the recoil lock is designed as a form-fitting shape block or as a force-fitting friction lock and in particular as a wrap spring lock or a clamp roller free-wheel.

16. Tape magazine according to one of the previous claims, **characterized in that** the storage unit (20) is screened from effects that interfere with the test tape (30) by a housing member (12).

17. Tape magazine according to one of the previous claims, **characterized in that** one housing wall (12a) forms an overlapping area (19) with another housing wall (13a), a passage (16) for the test tape (30) which is preferably provided with a sealing means (17) for sealing against the test tape (30) being formed along the overlapping area (19).

18. Hand-held device for analysing a body fluid and in particular a portable blood sugar measuring instrument with a tape magazine (10, 40, 50) according to one of the claims 1 to 17, which tape magazine is preferably replaceable.

## Revendications

1. Magasin-bande pour un appareil portatif (100) pour l'examen d'un fluide corporel comprenant une bande test (30), une unité de réserve (20) pour la bande test non utilisée et une unité à déchets (24) pour la bande test utilisée, l'unité à déchets (24) étant entraînée pour avancer la bande test (30), et des moyens de freinage (17, 28, 43, 51) intégrés, qui maintiennent la bande test (30) sous tension de traction, **caractérisé en ce que** l'unité à déchets (24) présente un blocage anti-retour contre un déroulement de bande intempestif.

2. Magasin-bande selon la revendication 1, **caractérisé en ce que** l'unité de réserve (20) est soumis à une force de freinage.

3. Magasin-bande selon la revendication 1 ou 2, **caractérisé en ce que** les moyens de freinage (17, 28, 43, 51) exercent une force de freinage directement sur la bande test (30).

4. Magasin-bande selon la revendication 1 ou 2, **caractérisé en ce que** les moyens de freinage (17, 28, 43, 51) s'appliquent sur un corps de bobine (21) pour la bande test (30), de sorte que la bande test est freinée indirectement.

5. Magasin-bande selon l'une quelconque des revendications 1 à 4, **caractérisé en ce que** les moyens de freinage (17, 28, 43, 51) exercent une force de freinage constante sur l'unité de réserve (20).

6. Magasin-bande selon l'une quelconque des revendications 1 à 5, **caractérisé en ce que** les moyens de freinage (17, 28, 43, 51) sont conçus comme élément de frottement.

7. Magasin-bande selon l'une quelconque des revendications 1 à 6, **caractérisé en ce que** les moyens de freinage (17, 28, 43, 51) présentent un ressort à lame (28) comme élément de frottement.

8. Magasin-bande selon l'une quelconque des revendications 1 à 6, **caractérisé en ce que** les moyens de freinage (17, 28, 43, 51) sont formés par un joint (17) pour l'étanchéité de l'unité de réserve (20) sur un passage (16) pour la bande test (30).

9. Magasin-bande selon l'une quelconque des revendications 1 à 4, **caractérisé en ce que** les moyens de freinage (17, 28, 43, 51) agissent avec une force de freinage variable sur l'unité de réserve (20).

10. Magasin-bande selon la revendication 9, **caractérisé en ce que** la force de freinage est variable en fonction du diamètre d'un bobinage de bande (23) de l'unité de réserve (20) ou en fonction de la force de traction de bande lors de l'avance.

11. Magasin-bande selon la revendication 9 ou 10, **caractérisé en ce que** les moyens de freinage (17, 28, 43, 51) présentent un levier d'inversion (43, 51) sollicité de préférence au moyen d'un ressort (41) avec une force de freinage, le levier d'inversion déviant la bande test (30) de préférence par un galet (46, 53).

12. Magasin-bande selon l'une quelconque des revendications 9 à 11, **caractérisé en ce que** les moyens de freinage (17, 28, 43, 51) présentent un mécanisme de compensation (54) qui est sollicité avec une force qui est variable en fonction du diamètre d'un bobinage de bande (23) de l'unité de réserve (20).

13. Magasin-bande selon la revendication 12, **caractérisé en ce que** le mécanisme de compensation est formé par une coulisse (54) sollicitée par ressort et touchant le bobinage de bande (23) côté pourtour, la charge du ressort diminuant avec la diminution du diamètre du bobinage de bande (23).

14. Magasin-bande selon l'une quelconque des revendications 1 à 13, **caractérisé en ce que** le blocage anti-retour est conçu sous forme d'un encliquetage de forme agissant par complémentarité de formes ou sous forme d'encliquetage de frottement agissant par adhérence de force, en particulier comme blocage à ressort enroulé ou roue libre à galet de serrage.

15. Magasin-bande selon l'une quelconque des revendications 1 à 14, **caractérisé en ce que** le blocage anti-retour s'engage dans une denture, par exemple dans le boîtier de bobine de l'unité à déchets, de sorte qu'une liaison mécanique par complémentarité de formes empêche dans le sens de marche opposé une rotation arrière de l'unité à déchets.

16. Magasin-bande selon l'une quelconque des revendications précédentes, **caractérisé en ce que** l'unité de réserve (20) est protégée par une partie de boîtier (12) contre des influences affectant la bande test (30).

17. Magasin-bande selon l'une quelconque des revendications précédentes, **caractérisé en ce qu'**une paroi de boîtier (12a) forme une zone (19) de chevauchement avec une paroi de boîtier (13a), un passage (16) doté de préférence d'un moyen d'étanchéité (17) pour l'étanchéité par rapport à la bande test (30) étant réalisé pour la bande test (30) le long de la zone de chevauchement (19).

18. Appareil rotatif pour l'examen d'un fluide corporel, en particulier un appareil portatif de mesure de taux de glycémie, équipé d'un magasin-bande (10, 40, 50) selon l'une quelconque des revendications 1 ou 17, qui peut de préférence être remplacé.
